# EUROPÄISCHE PATENTSCHRIFT

(11) **EP 0 983 247 B1**
(45) Veröffentlichungstag und Bekanntmachung des Hinweises auf die Patenterteilung: **22.01.2003**
(21) Anmeldenummer: 98930671.7
(22) Anmeldetag: 04.05.1998
(51) Int. Cl.: C07D 221/14, C07D 209/48

(54) **VERFAHREN ZUR HERSTELLUNG VON AROMATISCHEN BISIMIDEN**
METHOD FOR PRODUCING AROMATIC BISIMIDES
PROCEDE DE PRODUCTION DE BISIMIDES AROMATIQUES

(30) Priorität: 16.05.1997 DE 19720803
(43) Veröffentlichungstag der Anmeldung: 08.03.2000
(73) Patentinhaber: BASF AKTIENGESELLSCHAFT, 67056 Ludwigshafen (DE)
(72) Erfinder: VIERGUTZ, Wolfgang, D-67067 Ludwigshafen (DE); KOSER, Stefan, D-67061 Ludwigshafen (DE)
(74) Vertreter: Grünecker, Kinkeldey, Stockmair & Schwanhäusser Anwaltssozietät
(86) Internationale Anmeldenummer: EP9802621
(87) Internationale Veröffentlichungsnummer: WO98052924

(56) Entgegenhaltungen:
- EP-A- 0 540 975
- WO-A-93/12092
- WO-A-94/02466
- WO-A-96/25400

## Beschreibung

Die vorliegende Erfindung betrifft ein Verfahren zur Herstellung von aromatischen Bisimiden.

Cyclische Imide werden üblicherweise aus cyclischen Carbonsäureanhydriden und Ammoniak bzw. einem primären Amin hergestellt (Chem.Rev. 1970, 70, 439-469). Weitere Methoden sind die Reaktion von Dicarbonsäuren und Ammoniak bzw. primären Aminen bei hoher Temperatur (200°C) oder die Umsetzung von Diestern mit Ammoniak bzw. primären Aminen in Anwesenheit von Natriumethanolat. Des weiteren werden z.B. Maleinimide in verschiedenen Lösemitteln wie DMF, Dioxan oder Dimethylacetamid unter Zugabe von katalytischen Mengen von N-Methylmorpholin synthetisiert (JP 5 8096-066-A).

Zur Herstellung von aromatischen Bis-Imiden wie Bis-Naphthalimiden werden Naphthalsäureanhydride mit Polyaminen in Lösemitteln wie DMSO, DMF, THF oder Ethanol erwärmt (WO 94/02466). Die synthetisierten Bisnaphthalimide wurden chromatographisch gereinigt. J.H. Sun verwendete ebenfalls Ethanol als Lösemittel zur Herstellung von Bisnaphthalimiden (US 5,488,110). Diese Synthesemethode ist jedoch nicht in größerem Maßstab durchführbar, da die Produktreinigung mittels Säulenchromatographie zu aufwendig ist.

Es wurde nun ein Verfahren gefunden, mit dessen Hilfe Naphthalsäure- und Phthalsäureanhydride auf einfache Weise in ihre Bisimide umgewandelt werden können.

Gegenstand der Erfindung ist ein Verfahren zur Herstellung von Bisimiden der Formel I 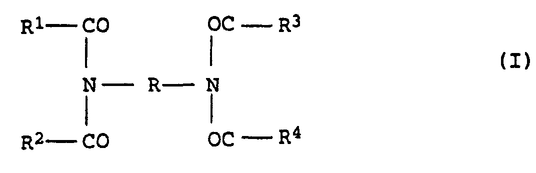 worin
R den Rest -Alk¹-NH-Alk²-(NH-Alk³)ₐ- bedeutet, worin Alk¹, Alk² und Alk³ C₂₋₆-Alkylenreste darstellen und a die Zahl 0 oder 1 ist,
R¹ + R² zusammen 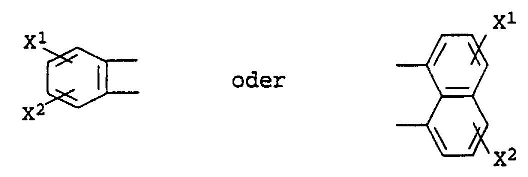 und
R³ + R⁴ zusammen 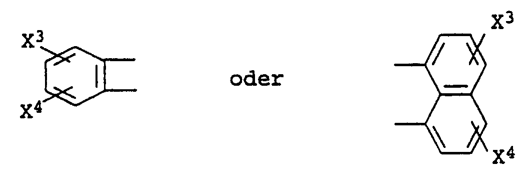 bedeuten, worin

X¹ und X², die gleich oder verschieden sein können, Wasserstoffoder Halogenatome, Nitrogruppen, gegebenenfalls durch 1 oder 2 C₁₋₄-Alkylreste substituierte Aminogruppen, Hydroxygruppen, Mercaptogruppen oder C₁₋₄-Alkylgruppen bedeuten und

X³ und X⁴, die gleich oder verschieden sein können, Wasserstoffoder Halogenatome, gegebenenfalls durch 1 oder 2 C₁₋₄-Alkylreste substituierte Aminogruppen, Hydroxygruppen, Mercaptogruppen oder C₁₋₄-Alkylgruppen bedeuten,
durch Umsetzen der entsprechenden Dicarbonsäureanhydride der Formeln II und III

R¹-CO-O-CO-R² (II)

R³-CO-O-CO-R⁴ (III),

worin R¹, R², R³ und R⁴ die oben angegebene Bedeutung haben, mit einem Amin der Formel H₂N-R-NH₂ , worin R dasselbe wie oben bedeutet,
dadurch gekennzeichnet, daß man die Umsetzung in Gegenwart eines tertiären Amins durchführt.

Als Ausgangsverbindungen der Formeln II und III kommen insbesondere gegebenenfalls substituierte Naphthalsäureanhydride in Betracht.

X¹ und X², die gleich oder verschieden sein können, sind vorzugsweise Wasserstoff-, Fluor- Chlor oder Bromatome, gegebenenfalls durch 1 oder 2 Methylreste substituierte Aminogruppen oder C₁₋₄-Alkylgruppen. Besonders bevorzugt ist X¹ ein Wasserstoffatom und X² ein Wasserstoffatom oder eine Aminogruppe.

Dasselbe gilt entsprechend für die Reste X³ und X⁴.

Die Reste X¹ - X⁴ stehen vorzugsweise in der m- bzw. p-Stellung an den Ringen. Die m-Stellung ist besonders bevorzugt.

Vorzugsweise sind X¹ und X³ sowie X² und X⁴ gleich.

Als Alkylengruppen Alk¹, Alk² und Alk³ kommen insbesondere solche mit 3 oder 4 Kohlenstoffatomen in Betracht. a ist vorzugsweise 0.

Als tertiäre Amine eignen sich für die Umsetzung: Triethylamin, Diisopropylethylamin, Dimethylcyclohexylamin und C₁₋₄-Alkyl-N-Morpholine. Unter diesen sind besonders die C₁₋₄-Alkyl-N-Morpholine uns speziell das N-Methyl-morpholin hervorzuheben.

Das tertiäre Amin dient in der Regel gleichzeitig als Lösemittel für die Umsetzung. Es ist aber auch möglich, weitere Lösemittel zuzusetzen wie beispielsweise sekundäre und tertiäre Alkohole mit bis zu 6, vorzugsweise bis zu 4 Kohlenstoffatome, Dioxan, Tetrahydrofuran oder Toluol. Bevorzugt ist das Arbeiten ohne zusätzliches Lösemittel.

Die Säureanhydride II werden mit den Aminen III in einem Molverhältnis von etwa 2:1 umgesetzt.

Die Menge an tertiärem Amin ist in einem weiten Bereich variabel. In der Regel wird so viel tertiäres Amin verwendet, daß sich die Reaktionspartner darin vollständig lösen. Man kommt jedoch auch mit geringeren Mengen an tertiärem Amin aus.

In der Regel beginnt man die Umsetzung bei 15-30°C und steigert die Temperatur langsam bis zur Rückflußtemperatur. Je nach der Geschwindigkeit der Erhöhung der Reaktionstemperatur ist die Umsetzung nach etwa 2 bis 4 Stunden beendet.

Das Reaktionsprodukt läßt sich aus dem Reaktionsansatz in einfacher Weise durch partielles Einengen des Reaktionsansatzes und anschließendes Ausfällen bei Temperaturen zwischen 0 und 5°C isolieren. Das so erhaltene Produkt zeigt bereits eine hohe Reinheit. Gewünschtenfalls kann das Produkt durch Umkristallisieren weiter gereinigt werden.

Das neue Verfahren besitzt gegenüber den bekannten Verfahren den Vorteil, daß es sich auch in technischem Maßstab sehr leicht durchführen läßt, wodurch die Verbindungen I sehr gut zugänglich werden.

Die Verbindungen I eignen sich beispielsweise zur Löschung der durch anionische optische Aufheller erzeugten Fluoreszenz. Die Naphthalimide zeigen cancerostatische Wirkungen.

### Beispiel 1

Bei Raumtemperatur wurden in einen 50L-Reaktor 1,89kg (11,75 Mol) bis-1,3-(2'-Aminoethyl)-propyl-1,3-diamin, 4,80kg (23,5 Mol, 97%ig) Naphthalsäureanhydrid und 29 L N-Methylmorpholin gegeben. Anschließend wurde die Temperatur innerhalb von 15 min auf 38°C erhöht. Nach 2 h Rühren bei dieser Temperatur wurde das Gemisch langsam auf die Rückflußtemperatur erhöht und 1 h bei dieser Temperatur gehalten. Danach wurden 5,8 L abdestilliert. Das Reaktionsgemisch wurde bei ca. 90°C filtriert und langsam auf 0 - 5°C abgekühlt. Der dabei ausgefallene Niederschlag wurde abfiltriert, mit eisgekühltem Methanol gewaschen und im Vakuum getrocknet. Die Ausbeute betrug 6,63 kg an N,N'-bis[2-(1,8-Naphthalimido)-ethyl]-1,3-diaminopropan, F: 160°C.

### Beispiel 2

Das Beispiel 1 wurde wiederholt, jedoch wurden die Reaktionspartner in einer Mischung aus 29 L N-Methylmorpholin und 5 L Ethanol umgesetzt. Die Ausbeute betrug 6,26 kg.

### Analog Beispiel 1 lassen sich folgende Verbindungen herstellen:

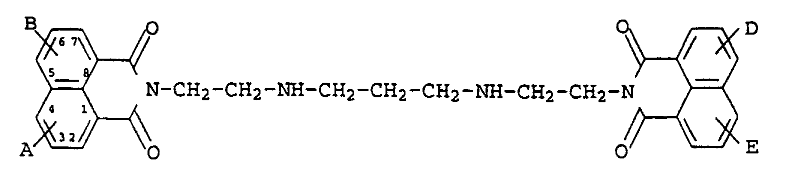

| Beispiel | A |
|---|---|
| 3 | 2-OH |
| 4 | 3-OH |
| 5 | 4-OH |
| 6 | 3-NO₂ |
| 7 | 4-NO₂ |
| 8 | 2-CH₃ |
| 9 | 3-Br |

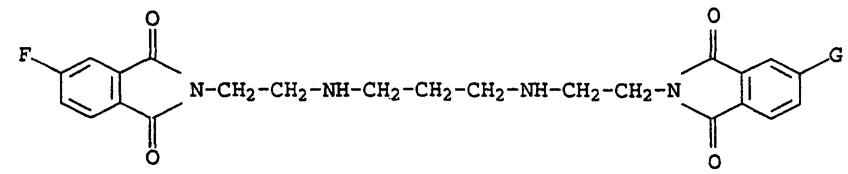

| Beispiel | F | G |
|---|---|---|
| 10 | H | H |
| 11 | H | t-C₄H₉ |
| 12 | F | F |

## Patentansprüche

1. Verfahren zur Herstellung von Bisimiden der Formel I 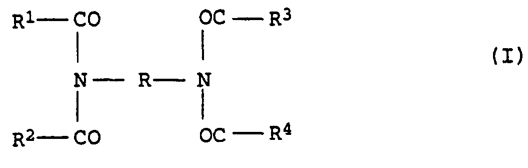 worin
R den Rest -Alk¹-NH-Alk²-(NH-Alk³)ₐ- bedeutet, worin Alk¹, Alk² und Alk³ C₂₋₆-Alkylenreste darstellen und a die Zahl 0 oder 1 ist,
R¹ + R² zusammen 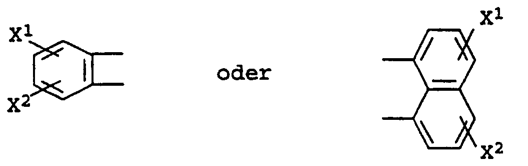 und
R³ + R⁴ zusammen 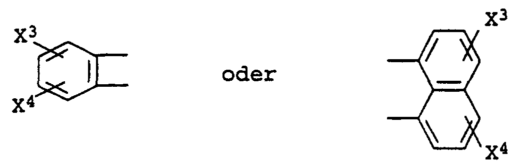 bedeuten, worin
X¹ und X², die gleich oder verschieden sein können, Wasserstoff- oder Halogenatome, Nitrogruppen, gegebenenfalls durch 1 oder 2 C₁₋₄-Alkylreste substituierte Aminogruppen, Hydroxygruppen, Mercaptogruppen oder C₁₋₄-Alkylgruppen bedeuten und
X³ und X⁴, die gleich oder verschieden sein können, Wasserstoff- oder Halogenatome, gegebenenfalls durch 1 oder 2 C₁₋₄-Alkylreste substituierte Aminogruppen, Hydroxygruppen, Mercaptogruppen oder C₁₋₄-Alkylgruppen bedeuten,
durch Umsetzen der entsprechenden Dicarbonsäureanhydride der Formeln II und III
R¹-CO-O-CO-R² (II)
R³-CO-O-CO-R⁴ (III),
worin R¹, R², R³ und R⁴ die oben angegebene Bedeutung haben, mit einem Amin der Formel H₂N-R-NH₂, worin R dasselbe wie oben bedeutet,
**dadurch gekennzeichnet, daß** man die Umsetzung in Gegenwart eines tertiären Amins durchführt.

2. Verfahren gemäß Anspruch 1, **dadurch gekennzeichnet, daß** man die Umsetzung in einem C₁₋₄-Alkyl-N-Morpholin durchführt.

3. Verfahren gemäß Anspruch 1, **dadurch gekennzeichnet, daß** man die Umsetzung in N-Methyl-morpholin durchführt.

## Claims

1. Process for the preparation of bisimides of the formula I 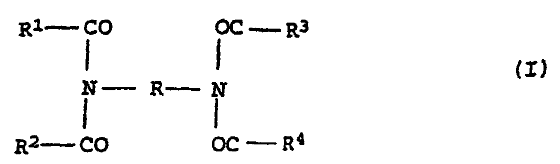 wherein
R denotes the radical -alk¹-NH-alk²-(NH-alk³)ₐ-, wherein alk¹, alk² and alk³ represent C₂₋₆-alkylene radicals and a is the number 0 or 1,
R¹ + R² together denote 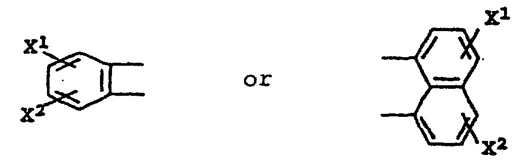 and
R³ and R⁴ together denote 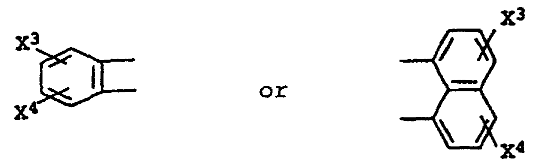 wherein
X¹ and X², which can be identical or different, denote hydrogen or halogen atoms, nitro groups, amino groups which are optionally substituted by 1 or 2 C₁₋₄-alkyl radicals, hydroxyl groups, mercapto groups or C₁₋₄-alkyl groups and
X³ and X⁴, which can be identical or different, denote hydrogen or halogen atoms, amino groups which are optionally substituted by 1 or 2 C₁₋₄-alkyl radicals, hydroxyl groups, mercapto groups or C₁₋₄-alkyl groups,
by reaction of the corresponding dicarboxylic acid anhydrides of the formulae II and III
R¹-CO-O-CO-R² (II)
R³-CO-O-CO-R⁴ (III)
wherein R¹, R², R³ and R⁴ have the meaning given above, with an amine of the formula H₂N-R-NH₂, wherein R denotes the same as above,
**characterized in that** the reaction is carried out in the presence of a tertiary amine.

2. Process according to claim 1, **characterized in that** the reaction is carried out in a C₁₋₄-alkyl-N-morpholine.

3. Process according to claim 1, **characterized in that** the reaction is carried out in N-methyl-morpholine.

## Revendications

1. Procédé de préparation de bisimides de formule I 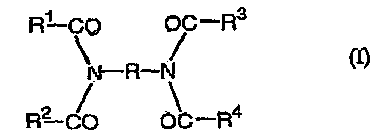 dans laquelle
R signifie le radical -Alk¹-NH-Alk²-(NH-Alk³)ₐ-, où Alk¹, Alk² et Alk³ représentent un radical alkylène en C₂₋₆ et a est un nombre égal à 0 ou 1,
R¹ + R² signifient ensemble 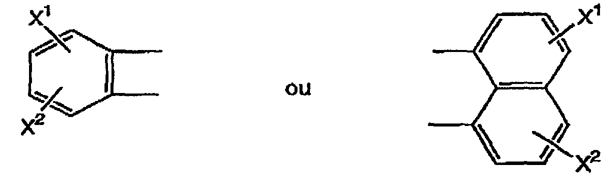 et
R³ + R⁴ signifient ensemble 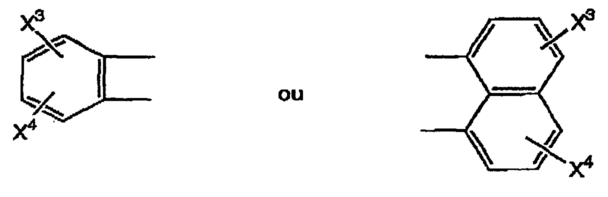 où
X¹ et X², qui peuvent être identiques ou différents, signifient des atomes d'hydrogène ou d'halogène, des groupes nitro, des groupes amino éventuellement substitués par 1 ou 2 radicaux alkyle en C₁₋₄, des groupes hydroxy, des groupes mercapto ou des groupes alkyle en C₁₋₄ et
X³ et X⁴, qui peuvent être identiques ou différents, signifient des atomes d'hydrogène ou d'halogène, des groupes amino éventuellement substitués par 1 ou 2 radicaux alkyle en C₁₋₄, des groupes hydroxy, des groupes mercapto ou des groupes alkyle en C₁₋₄,
par réaction des anhydrides d'acides dicarboxyliques correspondants de formules II et III
R¹-CO-O-CO-R² (II)
R³-CO-O-CO-R⁴ (III)
dans lesquelles R¹, R², R³ et R⁴ ont les significations données ci-dessus, avec une amine de formule H₂N-R-NH₂, où R a la même signification que ci-dessus,
**caractérisé en ce qu'**on effectue la réaction en présence d'une amine tertiaire.

2. Procédé selon la revendication 1, **caractérisé en ce qu'**on effectue la réaction dans une alkyl(en C₁₋₄)-N-morpholine.

3. Procédé selon la revendication 1, **caractérisé en ce qu'**on effectue la réaction dans la N-méthyl-morpholine.
